# EUROPEAN PATENT SPECIFICATION

(11) **EP 0 760 007 B1**
(45) Date of publication and mention of the grant of the patent: **18.07.2007**
(21) Application number: 95919156.0
(22) Date of filing: 09.05.1995
(51) Int. Cl.: C12Q 1/68

(54) **HIGH RESOLUTION MAPPING METHODS AND MAPPING PROBES**
HOCHAUFLÖSENDE GENKARTIERUNGS VERFAHREN UND GENKARTIERUNGS SONDEN
PROCEDES DE CARTOGRAPHIE HAUTE RESOLUTION ET SONDES DE CARTOGRAPHIE

(30) Priority: 13.05.1994 US 242553
(43) Date of publication of application: 05.03.1997
(73) Proprietor: Cedars-Sinai Medical Center, Los Angeles, California 90048-1865 (US)
(72) Inventor: KORENBERG, Julie R., Los Angeles, CA 90046 (US); CHEN, Xiao-Ning, Monterey Park, CA 91754 (US)
(74) Representative: Fiener, Josef
(86) International application number: PCT/US1995/005918
(87) International publication number: WO 1995/031573

(56) References cited:
- TRENDS IN BIOTECHNOLOGY, vol. 10, 1992 CAMBRIDGE GB, pages 27-32, KORENBERG ET AL. 'USING FLUORESCENCE IN-SITU HYBRIDIZATION FISH IN GENOME MAPPING.'
- SCIENCE, vol. 247, no. 4938, 5 January 1990 pages 64-69, XP 000371628 LICHTER P ET AL 'HIGH-RESOLUTION MAPPING OF HUMAN CHROMOSOME 11 BY IN SITU HYBRIDIZATION WITH COSMID CLONES' cited in the application
- PROCEEDINGS OF THE NATIONAL ACADEMY OF SCIENCES OF USA, vol. 87, 1990 WASHINGTON US, pages 6639-6643, CHERIF ET AL. 'Simultaneous localisation of cosmids and chromosome R-banding by fluorescense microscopy' cited in the application
- PROCEEDINGS OF THE NATIONAL ACADEMY OF SCIENCES OF USA, vol. 87, 1990 WASHINGTON US, pages 9358-9362, ROWLEY ET AL. 'Mapping chromosome band 11q23 in human acute leukemia with biotinylated probes' cited in the application
- CYTOGENET. CELL. GENET., vol. 59, 1992 pages 311-312, LEMIEUX ET AL. 'A simple method for simultaneous R- or G-banding and FISH of small single copy genes' cited in the application
- CHROMOSOMA, vol. 102, 1993 pages 325-332, HENG ET AL. 'Modes of DAPI banding and simultaneous in situ hybridisation' cited in the application
- GENOMICS, vol. 9, 1991 SAN DIEGO, US, pages 770-774, BALDINI ET AL. 'In situ hybridisation banding of human chromosomes with Alu-PCR products' cited in the application
- AMERICAN J. OF HUMAN GENETICS, vol. 51, no. 4, October 1992 CHICAGO, US, page 443 KORENBERG ET AL. 'Human cDNA mapping using FISH'
- ANALYTICAL BIOCHEMISTRY, vol. 217, March 1994 NEW YORK US, pages 205-209, ROUQUIER ET AL. 'Application of bacterial artificial chromosomes to the generation of contiguous physical maps: a pilot study of human ryanodine receptor gene (RYR1) region. '
- PROCEEDINGS OF THE NATIONAL ACADEMY OF SCIENCES OF USA, vol. 89, 1992 WASHINGTON US, pages 8794-8797, SHIZUYA ET AL. 'CLONING AND STABLE MAINTENANCE OF 300-KILOBASE-PAIR FRAGMENTS OF HUMAN DNA IN ESCHERICHIA-COLI USING AN F-FACTOR-BASED VECTOR.'
- HUMAN GENETICS, vol. 69, 1985 pages 300-303, MAGENIS ET AL. 'Localisation of the beta-globin gene to 11p15by in situ hybridisation: utilisation of chromosome 11 rearrangements' cited in the application
- CYTOGENET. CELL GENET., vol. 69, March 1995 pages 196-200, KORENBERG AND CHEN 'Human cDNA mapping using a high resolution R-banding technique and FISH'

## Description

### ACKNOWLEDGEMENT

This invention was made in part with Government support under Grant No. DE-FG03-92ER-61402, from the Department of Energy, and under Grant No. R29HG00037-04, from the National Institutes of Health Human Genome Initiative. The Government may have certain rights in this invention.

### FIELD OF THE INVENTION

The present invention relates to methods and reagents used to identify genes responsible for human disease. The present invention also relates to construction of high resolution genetic maps spanning the human genome.

Throughout this application various publications are referenced within parentheses.

### BACKGROUND OF THE INVENTION

The resources provided by the Human Genome Project are shaping the strategies used to identify the 50-100,000 genes responsible for human disease. Current efforts to rapidly and cost-effectively map genes responsible for genetic disease using highly polymorphic markers (Weissenbach, J. et al., Nature 359:794-801 1992) have exceeded the ability to relate the available markers to the 10,000 or more human cDNAs now known. At present, fragments of approximately 20,000 of a total of about 50,000-100,000 predicted human genes have been cloned and in part sequenced. Despite these successes, only about 3000 of these clones have been mapped to their respectve chromosomal sites, mostly to large regions of 10-20 Mb which include multiple bands. Thus, a significant weakness of current mapping techniques derives from the inability to identify evenly spaced highly informative markers along the chromosome.

Physical mapping techniques provide a complement to genetic linkage. For example, standard agarose gel electrophoresis gives resolution in the 1 to 10 kb range. Pulsed field techniques extend this range to the megabase level. However, gel electrophoresis methods are not useful for the initial localization of a DNA sequence that has not been mapped previously. Furthermore, such methods do not directly provide the ability to order DNA sequences which are more than a few hundred kilobases apart, nor do such methods provide the ability to assign DNA sequences to specific chromosomal regions.

Chromosome banding techniques have facilitated the identification of specific human chromosomes and presently provide the major basis upon which chromosomal aberrations are diagnosed. For initial chromosome assignment and gene localization, several techniques have been used to obtain a high level of precision in localization. These techniques, however, have some disadvantages. For example, techniques for banding that depend in part on DNA loss after UV irradiation, may therefore result in some signal loss (Cherif et al. in Proc. Natl. Acad. Sci. USA 87:6639-6643 (1990); and Takahashi et al. in Hum. Genet 86:14-16 (1990)). In contrast to other techniques, Standard DAPI (4',6-diamidino-2-phenylindole), or PI (Propidium Iodide) staining, produce inconsistent patterns and are, in general, of lower resolution (Pinkel et al. in Proc. Natl. Acad. Sci. USA 83:2934-2938 (1986); Lemieux et al. in Cytogenet Cell Genet. 59:311-312 (1992); Yamada et al. in Genomics 15:449-452 (1993); and Heng et al. in Chromosoma 102:325-332 (1993)). Other techniques produce strong well-differentiated patterns, suitable for detection of large probes with strong signals, but may obscure weak signals produced by small probes (Rowley et al. in Proc. Natl. Acad. Sci. USA 87:9358-9362 (1990)). Finally, Giemsa staining after hybridization, typical of other staining techniques, necessitates two rounds of microscopic evaluation, one to detect the hybridization probe and the other to localize the signal on banded chromosomes (Klever et al. in Hum. Genet. 86:484-486 (1991)). Methods using simultaneous hybridization with interspersed repeated sequences to mark bands may also be used (Baldini et al. in Genomics 9:770-774 (1991)), but often prove difficult to obtain metaphases that show both clear banding patterns and hybridization signals produced from small probes.

The most direct method for identifying the chromosomal locus of a segment of human DNA is by *in situ* hybridization. Although unique sequences less than 1 Kb long can be localized by using isotopically labeled probes, autoradiographic development times are long (often weeks or months), extensive statistical analysis is required, and the mapping precision is limited by the necessity of having to capture the emitted isotopic signal by an emulsion overlay. In contrast, nonisotopically labeled probes, while offering improved speed and spatial resolution, suffer from a lack of sensitivity.

Trends in Biotechnology, vol.10, 1992, pp. 27-32, discloses a method for high resolution mapping of (a) nucleic acid sequence (-s) to a single band on a human chromosome, by means of FISH of biotinilated probes and post FISH counterstaining with two counterstains to yield high resolution R bands wherein the counterstains are applied in two staining rounds, first one with chromomycin A3 and then (sequentially) one with distamycin A.

Thus, although a number of techniques have been described for chromosome band assignment, each of these prior art techniques suffer from drawbacks. For example, most prior art techniques are of lower resolution than the standard Giemsa banding, utilize fluorescent dyes whose spectra overlap with the standard FISH tags, result in DNA loss, or may be used only for large genomic probes inserted in cosmids or phage vectors.

In spite of the above-described developments in mapping techniques, efforts to rapidly, efficiently, and accurately map a large number of clones to develop a regularly spaced array of cloned genomic sequences covering the human genome, therefore, have been impeded by various limitations encountered at several levels. As a result, the development of detailed maps of specific chromosome band regions defined by unique markers, particularly in situations where deletions, translocations, or duplications define genes of medical significance, is currently unattainable.

Thus, a need exists to develop methods and reagents that facilitate the accurate assignment of genes to loci on single chromosome bands. Such developments will expedite correlation of gene sequencing information with the biological significance thereof, and hence facilitate identification of suitable medical treatments. The present invention satisfies these needs and provides related advantages as well.

### SUMMARY OF THE INVENTION

In accordance with the present invention, high resolution, rapid and reproducible chromosomal banding methods have been developed. Invention methods enable the accurate mapping of genes to single chromosome bands with no registration shift.

The present invention relates to a method for mapping cDNA sequences as defined in claim 1 and to the use of reagents as defined in claim 4.

### BRIEF DESCRIPTION OF THE FIGURE

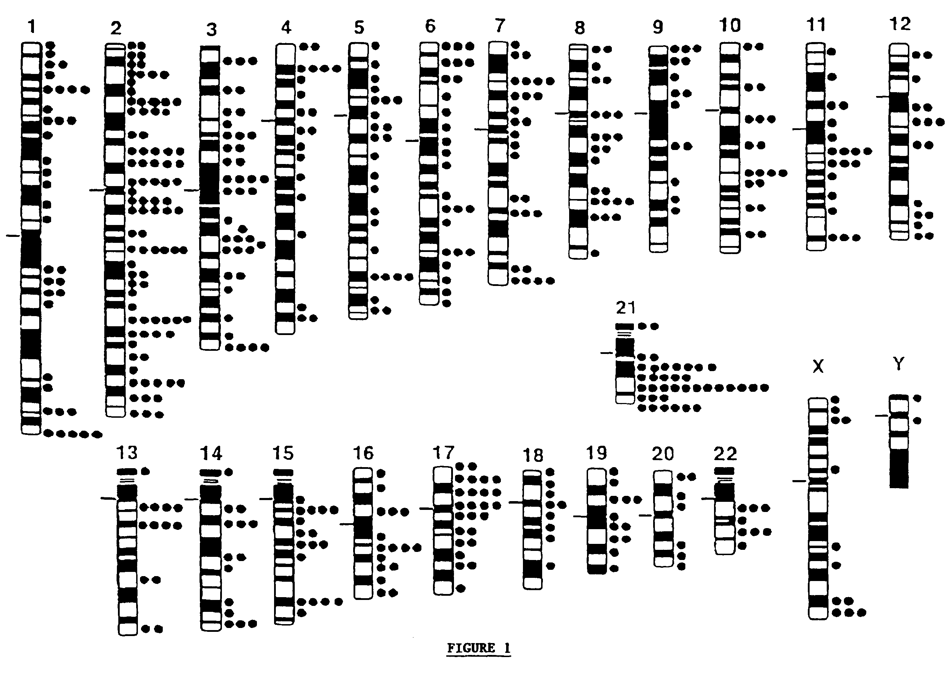

Figure 1 illustrates the single chromosome band mapping strategy of the present invention. The solid dots to the right of each chromosome band represent unique molecular cytogenetic reagents that identify and mark specific locations on a chromosomal band.

### DETAILED DESCRIPTION OF THE INVENTION

The present invention provides a high resolution, reproducible reverse (R) banding method that enables rapid determination and accurate assignment of single chromosome band map positions for gene sequences. Invention methods expedite correlation of gene sequences having biological significance with specific chromosomal band locations. The data generated by invention methods enable construction of complete physical maps of all chromosomes in the human genome and, hence, identification of suitable medical treatments for genetic disorders. Additionally, the improved resolution provided by invention methods allows for accurate establishment and evaluation of mouse homologies relative to human genetic disease. The teachings of the present invention enable accurate detection of a transcribed locus within a multigene or processed pseudogene family containing homologies of approximately 93% at the nucleotide level.

Fluorescence in situ hybridization (FISH) is one of the most accurate and rapid methods of assigning a single gene to a specific human chromosome band and is an essential element in both human gene mapping and in clinical cytogenetics. (Pinkel, D. et al., Proc. Natl Acad. Sci, USA, 83:2934 (1986)). The present invention provides an improved combination of sensitive fluorescence *in situ* hybridization with high-resolution chromosome banding to map cDNAs with an average insert size of <2 Kb to single human chromosome bands. Also provided are methods for producing high-resolution R-banding compatible with in situ hybridization.

In an alternative embodiment, the present invention provides methods for mapping cDNA. probes directly on R-banded human chromosomes. Also provided are specific methods for chromosome preparation, hybridization, and signal production that can be applied in combination with the R-banding method. Invention methods and reagents enable construction of complete physical maps of human chromosomes.

The unique reverse (R) banding of the present invention is achieved by post-in situ hybridization staining with chromomycin A3 and distamycin A. The distinct banding pattern obtained with invention hybridization method enables simultaneous visualization of both the fluorescent R bands and the in situ hybridization signals using either standard photomicroscopy or an automated image acquisition system. The sensitivity of this technique permits mapping of cDNAs in the size range of 1.2-3 Kb to single human chromosome bands using only standard photomicroscopy. The invention method is rapid and reproducibly reveals bands at the 350-700 stage.

An important issue in the practice of the present invention is the need to define criteria for confidence in mapping results derived from weak signals. As with any signal present in significantly less than 100% of chromosomes, conservative mapping criteria have been established to distinguish true signals from non-specific chromosomal background. Only signals that are seen on both chromatids of a single chromosome at the same band position were considered positive results. Further, as many metaphase chromosomes may overlap or be otherwise lost from the analysis, the signal on the second chromosome is not used to increase the mapping certainty. Thus, a probe is considered mapped when at least one chromosome is positive for signals at a single site in greater than or equal to 10% of 200 metaphase cells examined; a probe is considered to be preliminarily mapped when present in 5-10% of metaphase cells examined; and as possibly mapped when present in 2-5% of metaphase cells examined. A signal is considered as background when less than 1% of cells are positive. Many genes have been and can be accurately mapped with signals present in only 2-5% of cells, for example, as shown with glutaminase (<5% - see below), mapped using invention methodology to 2q32 (with a previous assignment of 2q32-34).

A comparison of invention R-banding patterns obtained before and after FISH revealed that invention methods, requiring staining after hybridization, do not alter the morphology of the chromosome. Chromosomes banding patterns of the "before" and "after" showed substantial similarity in resolution and clarity. This banding pattern is observed in greater than 90% of metaphases in a given preparation and is identical in chromosomes with or without bromodeoxyuridine (BrdU) incorporation. Modifications of the prior art banding technique, developed by Magenis et al. in Hum. Genet 69:300-303 (1985), were made to preclude the rapid fading typically encountered using the prior art technique. For example, the pH of the McIlavane's buffer (Schweizer, D. et al., Exp. Cell Res., 111:327 (1979)) was increased; the staining time with chromomycin A3 was varied according to probe size; and two rounds of chromomycin A3/distamycin staining were employed. Additionally, to photograph or acquire images of probes and banding patterns simultaneously, the exposure times and staining intensity were adjusted according to the signal strength. For smaller probes producing weak signals, the exposure time required when using standard photomicroscopy may vary from 15-200 seconds. In comparison, shorter times are required for recording more intense signals. All standard photomicroscopy utilized Kodak 100ASA film to maximize resolution. Faster film may be used to decrease exposure , time, however, at the sacrifice of band resolution.

Provided herein are methods for attaining high resolution banding patterns that are reproducible and can be successfully applied by skilled artisans as a routine technique for chromosomal band localization of cDNA sequences.

Currently, YACs, or yeast artificial chromosomes, are utilized for most mapping of the human genome. YACs permit cloning of fragments of ≥ about 500 Kbp. However, some difficulties have been encountered with the manipulation of YAC libraries. For example, in various YAC libraries, a fraction of the clones result from co-cloning events, i.e., they include in a single clone noncontiguous DNA fragments. A high percentage of YAC clones, particularly clones having high molecular weight inserts, are chimeric. Chimeric clones map to multiple sites on the chromosome and, thus, hamper the progress of mapping and analysis. Another problem endemic to YAC cloning is caused by DNA segments that are unclonable or unstable and tend to rearrange and delete.

Bacteria Artificial Chromosomes (BACs), provide an alternative to the YAC system. BACs mitigate the most problematic aspects of YACs such as, for example the high rate of chimerism and clonal instability. BACs are based on the *E. coli* single-copy plasmid F factor and are capable of faithful propagation of DNA fragments greater than about 300 Kb in size. BACs have a number of physical properties that make them amenable to physical mapping, including easy manipulation and an absence of chimerism. The lack of chimerism and the capacity to propagate large exogenous insert DNAs make the BACs excellent candidates for chromosome walking and the generation of contiguous physical maps.

Unlike YACs, which are linear, BACs with inserts exist as supercoiled circular plasmids in *E. coli.* This configuration permits easy isolation and manipulation of the large DNA in solution with minimal breaking, whereas yeast chromosomes are more difficult to isolate intact since the linear DNA is more susceptible to shear.

The BAC cloning system, as utilized herein, provides an intermediate physical mapping system that can be interspersed between YACs and cosmids. Although the YAC system remains a very powerful tool for large-scale mapping (since molecules larger than one megabase have been cloned), BACs are much easier to use than YACs and more amenable to fine physical mapping and chromosome walking. Indeed, the DNA segments that are unclonable or unstable in the YAC vector, i.e., regions where YACs have deletions or gaps, have been found to clone quite well in BACs. Furthermore, BACs are as easy to manipulate as cosmids, with an insert size that can be up to 10 times larger. BACs offer high cloning efficiency, easy manipulation of the cloned DNA, and stable maintenance of inserted DNA.

The present invention significantly increases and improves the potential of FISH. Invention techniques, therefore, enable those of skill in the art to rapidly produce high resolution maps of transcribed sequences.

The present invention also provides use of compositions as defined in claim 4 for unique single band chromosomal sites in the human genome (see Figure 1). Reagents are unique molecular cytogenetic markers that identify and mark specific locations on a chromosomal band. As used herein, reagent refers to a short stretch of DNA or cDNA sequence, readily detected, that uniquely identifies a physical genomic location. These reagents can be used as probes suitable for labeling and can be used to identify the precise chromosomal location of disease determining genes.

The advantages of the present invention over other methods can be illustrated, for example, by the mapping of over 900 DNA sequences including 130 cDNAs, 30 plasmids, 50 cosmids, 500 Bacterial Artificial Chromosomes (BACs) and 150 YACs to single human chromosome bands. Additionally, 39 newly cloned genes expressed in the human brain have been mapped, 26 of which define new loci with significant homology to known genes; 20 of these represent new members of multigene families in higher organisms, and 6 describe new members of genes related to those of lower organisms. They include genes related to phosphodiesterases, phosphatases, protein kinases, sodium channels, the Very Low Density Lipoprotein (VLDL) receptor, axonal glycoproteins, membrane transport proteins, members of the APP (amyloid protein precursor) family (one mapped to a region of previously suggested Alzheimer linkage, and the second providing a candidate locus for future studies), and the like. When combined with their map positions, as shown in Table 1, these genes provide immediate candidates for neurological diseases both in humans and in the homologous regions of the mouse.

**TABLE 1**

| Gene Name | Insert Size | Mapping Position |
|---|---|---|
| Putative APP-c100 | 11 Kb (*c) | Xq24 |
| Discs Large Tumor Suppressor | | 17p12-13.1 border |
| Human Folylpolyglutamatease | 2.1 Kb (c) | 9q34.1 |
| Fibulin | 2.2 Kb (c) | 22q13.3 |
| Sodium Channel 3 | 1.6 Kb (c) | 2q24 |
| VLDL receptor | 1.2 Kb (c) | 2q24 |
| Fibromodulin | 3 different sequences | 1q32 |
| Aggrecan | (c) | 15q26 |
| Tg737 | (c) | 13q12 |
| Argininosuccinate Synthetase | 1.5 Kb (c) | 9q34.1 |
| Cytosolic Serine | 2.0 Kb (c) | 11p11.2 |
| Hydroxymethyltransferases | | |
| Mitochondrial Serine Hydroxymethyltransferase | 2.0 Kb (c) | 12q13.2 |
| Potassium Channel | | 11p14-15 border |
| G Protein | | |
| CON E & Gα i3 | (c) | 1p13.1 |
| OD & Gα i2 | (c) | 3p21.3 |

| | | |
|---|---|---|
| *c= cDNA | | |

The results presented herein demonstrate the application of the present invention to large scale cDNA mapping. These results distinguish the present invention over other methods in accuracy (including high resolution chromosome band assignment, multiple signal acquisition and analysis), sensitivity and speed of mapping cDNA sequences.

For interpreting the significance of signals at multiple sites, one can assume that decreases in the target size or homology will result in a decrease in the proportion of cells or chromosomes with positive signals. This is particularly true for second-site hybridizations within the same cell or cell population in which the experimental variables are largely held constant. Therefore, for signals representing potential pseudogenes and multigene families in which target size is constant or more likely smaller, the site with the larger proportion of positive cells most likely represents the transcribed locus. For example, in multigene families with 93.5% and greater homology, a measure of the proportion of cells yielding positive signals is expected to define the transcribed locus.

The relationship between the percentage of positive cells and transcribed versus pseudogene loci was tested as described below. (see EXAMPLES 11-12). For most unprocessed pseudogenes, the signal is smaller because of the decreased pseudogene target size (due to truncation and rearrangement), and less probable, because of decreased homology, due to sequence drift, of the target versus the transcribed probe. For processed pseudogenes, the issues differ in that the homology may still be high but the genomic arrangement is different. A concern, for example, is that despite decreased sequence homology, a recently derived processed pseudogene, with clustered exons, may serve as a better hybridization target than the genomic locus of the transcribed gene in which introns interrupt the exon hybridization targets. This concern is inappropriately heightened by the well-known observation in Southern blots, that highly homologous pseudogene loci generate bands that are significantly more intense than those bands associated with the transcribed locus. Of course, in Southern blots, band density reflects both target size and sequence homology. Therefore, a pseudogene band detected with a cDNA for the related transcribed locus, may represent many more exons (of lower homology) than a similar-sized band from the transcribed locus, that may be divided into several less dense bands.

At the level of resolution of the metaphase chromosome, using fluorescence in situ hybridization, all exons are detected as a single spot. Therefore, sequence homology and exon target size (number of exons) are significant determinants of signal size. Other determinants, for example, include accessibility, salt concentration, base composition, fragment secondary structure, and the like. It is expected that, due to decreases in homology alone, most processed pseudogenes will generate smaller signals than those expected from the transcribed locus.

It is important to appreciate that the method of the present invention is, in some manner, a closer correlate of the traditional Cot curve than is solution hybridization to a fixed membrane. This is because each chromosome represents a single molecule, and the proportion of chromosomes with specific hybridization signals therefore sensitively reflects the probability of hybridization, i.e., the proportion of molecules hybridized at a given Cot. Although approximated somewhat by density, this information is largely lost in standard Southern blotting with non-linear signal density and multiple bands. Amplification of FISH signals does contribute to nonlinearity, in that the increase in size and intensity clearly contribute to detection. Because both the transcribed gene and possible pseudogene loci are detected in the same preparation, both probe concentration and hybridization conditions, viz, Cot, are held constant for the two and the proportion of cells with signals largely reflects the degree of homology and target size. Therefore, it may be expected that, given the sequence drift and rearrangements of pseudogenes, the proportion of cells with positive signals will be greater for the transcribed locus than the pseudogene.

The relationship of target to probe size is also important for specificity of the hybridization signal. For example, given that the probe and target are both larger than about 50-100 bp, (the size required for single hit stable hybridization under the conditions used), for equal target sizes (pseudogene and transcribed loci), the locus with the higher degree of homology and therefore stability, will generate the large signal. As the probe size increases with respect to the target size, as with small exons (<50-100 bp), a larger proportion of the probe is involved in imperfect fits. For example, for hybridized cDNA fragments containing more than one exon, the second exon will remain unpaired, potentially decreasing the stability of the perfect match. Therefore, large probe sizes (>200 bp) may tend to favor signal production from targets with contiguous exons where the exon size is small. This may not be a major contributor to signal where exons are larger than probe size. Therefore, the use of small probe size (50-200 bp) can facilitate the relative detection of the transcribed versus processed pseudogene locus when using the present invention as described.

The methodologies of the present invention maximize the difference in signal strength between closely related sequences and reproducibly map smaller fragments in the size range of cDNAs. Thus, invention methods differ from prior art methods in several ways, e.g., by the use of higher stringency conditions (including increased wash temperatures), multiple amplifications, and the use of smaller fragment probes in the 100-200 bp average range. The use of smaller fragment size and higher stringency washes tend to increase the specificity of hybrid formation allowing the sensitive definition of the transcribed locus with homology differences of as little as 6% as shown in the argininosuccinate synthase system. (see EXAMPLE 11).

The cDNA framework produced will both anchor and confirm the detailed YAC and genetic maps and immediately provide large numbers of mapped human genes which can be studied for their role in human genetic diseases.

The following examples are intended to illustrate, but not limit, the present invention.

### EXAMPLE 1

### Metaphase Preparation

Chromosomes were prepared by using a BrdU block, (Zabel et al. in Proc. Natl. Acad. Sci. USA 80:6932-6936 (1983)) with some modification. Briefly, human peripheral lymphocytes were grown for 72 hours at 37°C in RPMI 1640 (GIBCO BRL, Gaithersburg, MD) supplemented with L-glutamine (2mM), 15% fetal calf serum, penicillin (100 IU/ml), streptomycin (0.05mg/ml) and 0.02% phytohemagglutinin. The cells were blocked in S-phase by adding 5-bromodeoxyuridine (0.8mg/ml) for 16 hours. They were then washed once with HBSS (Hanks Balanced Salt Solution) (GIBCO BRL, Gaithersburg, MD) to remove the synchronizing agent and were released by incubating for five to six more hours in medium supplemented with 2.5µg/ml of thymidine. Cultures were harvested by the addition of 0.1µg/ml of colcemid for 10 minutes followed by 0.075 M KCl hypotonic solution for 15 minutes at 37°C prior to fixation with a 3:1 mixture of methanol and acetic acid, for 1-5 minutes.

### EXAMPLE 2

### High Quality Chromosome Preparation

To obtain high quality chromosome preparations, the metaphase spreads were prepared by letting one drop of suspension fall onto alcohol-cleaned slides, evenly and flat, completely free of cytoplasm. The slides were then placed above a container filled with heated water for 20-60 seconds depending on the ambient humidity. The optimum conditions were determined by first checking several slides under a phase contrast microscope. For best results, the slides should be aged at room temperature for at least 2-3 weeks prior to *in situ* hybridization. Slides are best stored at -70°C after aging. The day before denaturation, slides are removed from -70°C and kept overnight at 4°C. Slides are then left at room temperature for 1-2 hours before baking them at 55-60°C for 2 hours, followed by denaturation in 70% formamide (at 66-70°C). For best results, fresh slides are denatured at 66°C.

### EXAMPLE 3

### Probe Fragment Size for Mapping Small DNA Probes

The fragment size of a probe labeled by nick translation should be around 100-200 bp and the concentration of probe DNA in the hybridization mixture is in the range of 20-40 ng/µl (i.e., 200-400 ng/slide). This can increase non-specific binding, but generally will produce good signal noise ratio. For reducing non-specific background hybridization of repeat sequences, a small amount of CotI DNA should be used with cDNA probes (i.e., 1-3µg of CotI with 200-400 ng of probe DNA).

### EXAMPLE 4

### Biotinylation of Probes

DNA probes were labeled with biotin-14-dATP by nick translation (GIBCO BRL). Unincorporated nucleotides were separated by chromatography (Sephadex G-50). The mean fragment size of all DNA probes was set at around 200 bp, with a range of 100-600 bp, established by nondenaturing agarose gels.

### EXAMPLE 5

### In situ Hybridization

To obtain specific hybridization of single-copy sequences with small DNA probes, the FISH method described by Lichter et al. in Science 247:64-69 (1990) was modified. RNase treatment is unnecessary for slides made more than one year prior to their use. When necessary, RNase treatment consisted of 100µg/ml RNase, placed on slides for 20 minutes at 37°C, followed by dehydration through a cold ethanol series of 70%, 90% and 100%. The denaturation of slides was performed at 67-70°C in 70% formamide/2x SSC (0.15 M NaCl, 0.015 M Sodium Citrate) for 1-2 minutes (fresh chromosome preparations require a shorter time). The hybridization buffer (10µl) containing the biotinylated probes together with 3µg Cot 1 DNA and 7µg sonicated salmon sperm DNA, (the ratio of probe: Cot 1 DNA is 1:15-30; total DNA concentration at 1µg/µl) was placed on each slide. A coverslip was then applied and sealed with rubber cement, followed by overnight incubation in a humidified chamber at 37°C.

### EXAMPLE 6

### Detection of Probe Signals by Immunofluorescence

After overnight hybridization, the slides were washed at 44°C in 50% formamide (v/v)/2X SSC (4 x 5 minutes) and then at 50-60°C in 2X SSC (3 x 5 minutes). The rinsing time was shorter (3 x 2 minutes) for small probes (insert size <1.5 Kb). To increase the intensity of the fluorescence signal produced by smaller probes, it is necessary to reduce the stringency of post-hybridization washes (2X SSC, at 45-50°C). Slides were then incubated for 10-20 minutes at 37°C with 100µl of 4X SSC containing 3% bovine serum albumin (BSA) and 0.1% Tween 20 for blocking. The buffer was then replaced with FITC-Avidin (0.5 µg/ml in 4X SSC/1% BSA/0.1% Tween 20), for 30 minutes at 37°C. After three 5 minutes washes at 45°C in 2X SSC/0.1% Tween 20, the hybridization signal was amplified by the addition of a biotinylated goat anti-avidin antibody layer (5µg/ml) for 30 minutes at 37°C. The slides were preincubated in 5% goat serum/2X SSC/3% BSA/0.1% Tween 20 for 10-20 minutes prior to the amplification step to reduce immunological non-specific binding. After washing, a second layer of FITC-avidin was added to the slides as previously described. To increase the intensity of the hybridization signal, a second round of amplification can be applied as necessary. The slides were then washed in 2X SSC/0.1% Tween 20 three times at 45°C and briefly left to drain.

### EXAMPLE 7

### Chromosome R-Banding

To view the chromosome bands and fluorescence signals simultaneously, the dyes chromomycin A3 and distamycin A were used as counterstain (Schweizer in Chromosoma 58:307-324 (1976); Schweizer in Hum. Genet 57:1-14 (1981); and Magenis et al. in Hum. Genet 69:300-303 (1985)). Immediately following the last detection, the slides were rinsed briefly in McIlavane's buffer (pH 8.5-9.0) Schweizer et al., supra. (diluted 1:1 with distilled water) prior to staining. Subsequently, 100µl of chromomycin A3 (0.5mg/ml in 1/2 McIlavane's buffer pH 8.5-9.0) was placed on slides for 10 minutes - 1 hour at room temperature in the dark. The staining time required depends on the freshness of the slides; fresher slides need longer staining times, whereas aged slides may need only 10 minutes. After a first round of staining, the slides were rinsed for 1 minute in 1/2 McIlavane's buffer and the excess fluid was shaken off. This was followed by a second round of staining by placing 50µl of 0.1mg/ml distamycin A on the slide, incubating for 1-2 minutes at room temperature, followed by rinsing, as above.

If using DNA probes having small inserts (e.g., expected to produce faint signals), at this point, the slides can be mounted with a thin layer of antifade solution containing p-phenylenediamine in phosphate buffer (Johnson et al. in J. Immunol. Methods 43:349-350 (1981)).

Maximum banding resolution and minimal fading is obtained by two rounds of chromomycin A3 and distamycin A staining. In addition, the best banding results are obtained by staining the slides immediately following the detection step, but no later than 2-3 days. Slides are best viewed through the microscope immediately after staining, but no later than 3 days for small probes (1.0 Kb - 2.5 Kb) or 1-2 weeks for larger probes (>2.5-3 Kb). Only a light counterstain is needed before examining the slide under a fluorescence microscope. In fact, an advantage of this banding technique is that the counterstaining step can be repeated at any point after hybridization to make the chromosomes brighter, if necessary. Further, after image capture, using computerized systems, the banding pattern may be selectively bleached to more clearly view the FITC hybridization signal.

### EXAMPLE 8

### Microscopy and Photography

The slides were viewed with a Zeiss Axiophot 100 or Axiovert 135 fluorescence microscope (Zeiss, Inc., Thornwood, NY). The FITC and chromomycin A3 are both excited by using a 400-490nm band pass exciter, 460nm dichroic, and 470nm barrier (Zeiss filter set #05). The hybridized segments appear as bright green-blue or bright yellow-green spots, while the rest of the chromosome bands appear dim green. Kodak Technical pan (ASA100) film was used for black and white photographs. Color images were captured by a cooled-CCD camera (Photometrics CH 250, Photometrics Ltd., Tuscon, AZ) using the BDS (Biological Detection Systems, Pittsburgh, PA) imaging software.

### EXAMPLE 9

### cDNAs

cDNAs were obtained from standard minipreparation cultures (Sambrook, J. et al., Molecular Cloning, A Laboratory Manual, 2d Edition (Cold Spring Harbor 1989)). These were isolated and sequenced at random from a fetal brain library cloned in bluescript (Stratagene, La Jolla, CA). For FISH mapping, cDNAs containing inserts in the size range greater than 1 Kb were defined, and included a subset of those with database matches as shown in Tables II and III.

**TABLE 2. Localization of novel genes by FISH**

| Clone ID | % of cells (+) | Database Match | Match Quality | Insert Size | Map Location |
|---|---|---|---|---|---|
| HFBCA82 | 20 | Calmodulin-dep. protein kinase type II beta | Rat 98% aa sim, 107 res. condons 28-end | 2.2kb | 7p13 |
| HFBCD19 | ≤5 | Mouse dilute peptide | 100% identical; short (51bp match) | 1.6kb | 10q24.3 to q25.1 |
| HFBCH61 | ≤5 | Clathrin-assoc. protein AP47 | yeast 82%, 62 res.; mouse 95%, 104 res. | 20.kb | Xq13 |
| HFBCI88 | 25 | Large ERK kinase | exact human match | 3.0kb | 1p36.1 |
| HFBCJ51 | 8 | Glutaminase | Rat 80%, 34 res.; chimera/splice? | 2.0kb | 2q32 |
| HFBCP87 | 50 | cAMP-specific phosphodiesterase 2(PDE2) | human PDE2 | 1.9kb | 1P31 to P32 |
| HFBCX08 | 85 | Rab5-like | Human 92%, 91 res. (215 res, full length) | 2.4 kb | 12q13 |
| HFBDR29 | 8 | voltage-gated sodium channel II | exact human match | 0.6kb | 2q31 |
| HFBEM30 | ≤5 | very low density lipoprotein receptor / VLDL | exact human match | 1.6kb | 7p13 to p14 |
| HHCMD29 | 15 | hypothetical protein 3' to XPR2 in Yarrowia lipolytica | Yeast 65%, 88 res.; R=no match | 2.2kb | 15q22 |
| HHCME18 | 25 | alpha-enolase | exact human match | 1.5kb | 1p36.3 |
| HHCME40 | 10 | Rhodanese (thiosulfate sulfurtransferase) | Bovine 97% sim, 70 res. | 2.0kb | 22q13.1 |
| HHCME78 | ≤5 | nerve terminal protein, SNAP25 | exact human match | 2.3 kb | 5q11.2 |
| HHCMG18 | 40 | AMP deaminase | human AMPD2 | 1.8kb | 5q32 |
| HHCMH17 | 30 | Mitogen-activated kinase | exact human match | 1.8kb | 12q21 |
| HHCMH42 | 60 | Axonal glycoprotein TAG-1; f-polyT | exact human match | 2.Okb | 1q32 |
| HHCMH89 | 40-50 | Membrane transport family (NodI) | rhizobium 72%, 71 res. | 4.0kb | 6q21 |
| HHCPB41 | 55 | Protein phosphatase 2A beta subunit | Human 77% (DNA), 288bp | 1.9kb | 4p16.3 distal |
| HHCPF23 | 20 | Glial fibillary acidic protein | exact human match | 2.1kb | 17q21 |
| HHCPK77 | 12 | Lon protease-like protein | exact human match | 1.8kb | 19p13.2 |
| HHCPQ06 | 40 | inositol triphosphate kinase (IP3) | Human 80%, 65 res.; as: 177-460; 849bp | 2.8kb | 19q12 |
| HFBCJ87 | 55 | carboxypeptidase | 53% human, 350 res. | 2.5kb | 7p13 to p14 |

**TABLE 3. Confirmation/Refinement of Map Positions of Loci by FISH**

| Source | % of cells (+) | Gene Symbol / Gene Product | Insert Size (kb) | Previous or Predicted Map Location | Localization by FISH |
|---|---|---|---|---|---|
| ATCC (*HGM) | 85 | C3 / complement component 3 | 4.3 | 19p13.3-9p13.2 | 19p13.3 |
| | 70 | F8VWF / von Willebrand Factor | 2.9 | 12pter-12p12+ps | 12p13.1 |
| | 45 | HMGCR / 3-Hydroxy-3-methylglutaryl-CoA reductase | 4.3 | 5q13.3-5q14+ps | 5q13.3 |
| | 15 | ASS / Argininosuccinate synthetase | 1.5 | 9q34-9qter+ps | 9q34.1 |
| Venter et al. (#1) | 50 | MAP-1B / Neuraxin / microtubule-associated protein 1B | 1.8 | 5q13 | 5q13 |
| | 30 | Aldolase A | 2 | 16q22-16q24 | 16q22 (major) 16q11 (minor) |
| | 25 | alpha-Enolase | 1.5 | 1pter-1p36.3 | 1p36.3 |
| | 20 | Glial fibrillary acidic protein | 2.1 | 17q21 | 17q21 |
| ATCC (*HGM) | 85 | C3 / complement component 3 | 4.3 | 19p13.3-9p13.2 | 19p13.3 |
| | 70 | F8VWF / von Willebrand Factor | 2.9 | 12pter-12p12+ps | 12p13.1 |
| | 8 | Glutaminase | 2 | HSA 2q32-q34 | 2q32 |
| Venter et al. (#2) | 45 | Amyloid Precursor-like Protein 1/ APLP1 | 1.6 | 19q11-q13 | 19q12 |
| | 30 | Amyloid Precursor-like Proten 2/ APLP2 | 1.5 | 11q26 | 11q25 |
| Denny et al. | 10 | B37 / Human lymphocyte-specific pp52 gene | 1.7 | Cosmid clone: 11pter+ps | 11p15.5 |
| Wilkie et al. | 25 | G-alpha-16 / G-protein | 2.1 | 19p13 | 19p13.3 |
| | 35 | G-alpha-i2 | 1.34 | Mouse 9, Predict Human 3 | 3p21.2 |
| | 20 | G-alpha-i3 | 2.4 | Mouse 3, Predict Human 11 | 11p13.3 |
| Cohn et al. | 25 | PHN-02 / human IL-1 alpha | 1.6 | 2q close to alphacentromere | 2q13.1 |
| ATCC (*HGM) | 85 | C3 / complement component 3 | 4.3 | 19p13.3-9p13.2 | 19p13.3 |
| | 70 | F8VWF / von Willebrand Factor | 2.9 | 12pter-12p12+ps | 12p13.1 |
| Shane et al. | 40 | h-FPGS / human folylpolyglutamate synthetase | 2.1 | 9 (cell fusion study) | 9q34 |
| Ingram et al. | 15 | VM1732 / Sodium Channel I alpha | 1.6 | YAC clone & PCR hybrids | 2q24 |
| Guellaën et al. | 30 | Glutamate dehydrogenase | 3 | 10q23.3 | 10q22-23 border |
| | 50 | D7S8 locus DNA (telomere) | 1.4 | 7q31 | 7q31 distal |
| | 40 | Membrane-assoc. protein HEM | 1.8 | 12q13.1 | 12q13.1 |
| | 65 | Coagulation Factor XIIIa | 2.8 | 6p25 | 6p25 |
| | 30 | ornithine Aminotransferase | 1.8 | 10q26 | 10q25-26 border |

### EXAMPLE 10

### Mapping Single Genes

The accuracy of the present invention was tested on the ability to map single genes with known map assignment and genes known to be members of multigene or pseudogene families. First, the map positions of 15 genes with previously known or predicted map positions have been confirmed and, in all cases refined considerably based on FISH analysis of cDNA clones. Further issues concerning single genes and the sensitivity of their detection are considered below.

For multigene families, accuracy in mapping the site of transcribed genes is determined by the target size, degree of homology, and potential genomic rearrangement. Mapping of a multigene family consisting of 15 G-protein α-subunits (Wilkie et al. in Nature Genetics 1:85-90 (1992)) was performed with this technology, and all human cDNAs were independently mapped to regions predicted by known murine homologies. Further, no second sites were observed with any cDNA, despite nucleotide sequence homology as high as 75% between Gαi2 and Gαi3.

For the mapping of Aldolase A, using a 2.0 Kb fragment, not only was the resolution increased but no secondary signals were detected at the known site of the related gene for Aldolase C, that is approximately 74% homologous over the entire coding region (Costanzo et al., 1988). These data suggest that for sequences of similar genomic arrangement in multigene families, unique map positions can be defined using invention methods even in the presence of nucleotide homologies of about 75% (higher than that of most members of known multigene families), and corresponding in general to protein homologies in the range of about 90%.

### EXAMPLE 11

### Detection of Pseudogene signals

As a stringent test of the potential preferential detection of signals from transcribed versus a processed pseudogene locus, a cDNA for the transcribed gene, argininosuccinate synthetase (AS), associated with a family of 14 processed pseudogenes was used to illustrate the ability of the present invention to distinguish between signals. Three of the pseudogenes have sequence homologies (versus the expressed sequence) of 93.5% (AS1p1), 92.9% (ASp3) and 89.4% (ASp7) over 2139 bp. Despite this homology, hybridization using a 1.5 Kb cDNA for AS revealed the highest proportion of 200 cells, (15%) of chromosomes with signals at 9q34.1, the transcribed locus, and secondary sites positive in 3% (chromosome 6p21) and 1% (7q32) of cells, defining the presumed pseudogene loci.

### EXAMPLE 12

### Detection of Transcribed Locus

An interesting example of detecting the transcribed locus in the presence of associated pseudogenes is noted in the mapping of the cDNA for pp52, a human lymphocyte-specific gene associated with four distinct genomic loci with more than 90% homology in one exon, but three of which are probable pseudogenes (May et al., 1993). Although cosmids for the related loci recognized more than one locus, some with close to equal frequency, the cDNA probe generated signal predominantly at chromosome 11p15.5, the transcribed locus.

### EXAMPLE 13

### Localization of Amyloid 4-like Genes

The potential relationship of the Amyloid precursor protein-like gene (APLP1) mapping in 19q13.1 to the late onset form of familial Alzheimer's disease (AD2) is of interest. Mutations of the amyloid protein precursor gene (APP) on chromosome 21 are responsible for one form of familial Alzheimer's disease, AD1. Evidence for linkage between AD2 and several DNA markers on proximal 19q has been obtained by Pericak-Vance et al., Cytogenet. Cell Genet., 58:751 (1991). In these families, using affected only in the analysis, a maximum multipoint LOD score of 4.4 was obtained near ATP1A3 (19q13.2) and D19S13 (19cenq13.1). Although increased dosage of the gene for apolipoprotein E, allele APOE4, located in 19q13.2, (telomeric to APLP1) is correlated with an increased risk for AD2, it is not unreasonable to consider, as suggested by Wasco et al., Genomics, 15:237 (1993) that a part of this risk association may be due to linkage disequilibrium with another gene, perhaps the APLP1 gene we have mapped in the region.

The second APLP gene (named CDE-1-BP because it binds to a yeast centromeric consensus sequence), maps to 11q26 and is also of interest but, perhaps less so with respect to familial Alzheimer disease, because, despite the higher homology to APP, there is nothing at present to suggest a genetic link. Nonetheless, both of these map positions provide candidate loci for evaluation of genes responsible for Familial Alzheimer Disease in families that are unlinked to either chromosome 21 or chromosome 19 (Swedish families: Lannfelt et al. in Nature Genetics 4:218-219 (1993); and Volga German families: Schellenberg et al.).

### EXAMPLE 14

### Localization of Protein Phosphatase 2A, beta subunit

There is considerable evidence that protein phosphatase 2A plays a role in a broad spectrum of cellular processes. These include the regulation of major metabolic pathways, translation, transcription, the G2-M cell cycle transition, and tumorigenesis (reviewed in Jones et al. in Cytogenet Cell Genet 63:35-41 (1993)). Therefore, the assignment of its map position to chromosome 4p16.3 provided an immediate gene candidate for a potential role in the Wolf-Hirschhorn syndrome (WHS) (characterized by profound growth and mental retardation). cDNA mapping was applied to the assignment of protein phosphatase within the 2.5 Mb critical region deleted on 4p16.3 (Gandelman et al., 1992).

### EXAMPLE 15

### Localization of Axonal Glycoprotein (TAG-1/axonin-1)

In recent years, cell-surface proteins such as TAG-1 have been identified that are expressed in neural tissue and are thought to play crucial roles in the development of neuronal networks. Human TAG-1/axonin has now been independently cloned and its expression found to be restricted to regions of neuronal differentiation in the developing nervous system (Hasler et al. in Biochem 211:329-339 (1993)). Therefore, the mapping of human TAG-1/axonin to chromosome band 1q32 provides a gene candidate of interest with respect to the Van der Woude syndrome (cleft-lip/palate with mucous cysts of the lower lip), caused by a deletion of this region.

### EXAMPLE 16

### Localization of Rhodanese

As a nuclear encoded gene for a mitochondrial protein, the map position of this gene on chromosome 22q13.1 now provides the basis for investigation of its relationship to mitochondrial diseases inherited in an autosomal dominant fashion. The mouse mutations mapping in this region are neurological and include gray tremor, waggler and blindness, all of potential interest with respect to this gene.

### EXAMPLE 17

### Localization of Inositol 1,4,5 triphosphate kinase

Maps in the chromosome 19 region linked to central core disease (CCO) of muscle are of interest (Mulley et al., Am J of Hum Genet 52:398-405 (1993)). The current linkage and physical data may immediately be applied to evaluate this relationship.

### EXAMPLE 18

### Loci of Calmodulin-dependent protein kinase type II beta

Although no candidate diseases occur in the region of chromosome 7p13, this gene is the second or third protein kinase (PRKAR1B), in addition to a calmodulin-like sequence, to be identified in this region.

### EXAMPLE 19

### Localization of Nod1 Membrane transport superfamily

This gene has high homology (72%) to the Rhizobium gene involved in the nodulation process and strong homology to ATP-dependent bacterial transport proteins. It provides one of the few known genes in the region of 6q21, along with two other neural receptors. Clearly genes with such a high degree of sequence conservation are likely involved in important functions.

### EXAMPLE 20

### Localication of Polymyxin B Resistance Protein

This gene, a probable protein kinase with very high homology to the yeast, maps in 12q21, and provides one of the first known genes in this region.

### EXAMPLE 21

### Localization of AMP deaminase

This gene plays a critical role in energy metabolism, maps in 5q32, a human region with receptors and homology to a mouse region in which neurological mutations are found including vt (vestigial tail with panhypopituitarism), dt (dwarf mutation).

### EXAMPLE 22

### Localization of cAMP-specific phosphodiesterase

Homologous to a previously defined human gene, this new gene, mapping in 1p31-32, a gene rich region, is the first mapped of its class.

### EXAMPLE 23

### Localization of XPR2

Alkaline extracellular protease, localized in 15q22, represents the first gene mapped in the entire band. This nucleates the region, provides the first extracellular protease mapped and may be of interest with respect to the 15q22-associated chromosomal rearrangement associated with promyelocytic leukemia.

## Claims

1. A method for high resolution mapping of a cDNA sequence to a single band locus on a human chromosome, wherein said method comprises fluorescence in situ hybridization and reverse (R) banding, said method comprising the steps of:
(a) in situ hybridizing human chromosome spreads that are aged at room temperature for at least 2-3 weeks prior to in situ hybridization with said cDNA sequence, wherein said cDNA sequence is biotinylated,
(b) contacting hybridized chromosome spreads with at least one fluorescent label,
(c) performing a counterstaining by post-in situ hybridization staining with chromomycin A3 and distamycin A, and
(d) detecting the presence of said cDNA sequence and determining the chromosomal band position of said cDNA sequence, wherein said cDNA sequence is considered mapped when at least one chromosome is positive for signals at a single site in greater than or equal to 10% of 200 metaphase cells examined; wherein said cDNA sequence is considered to be preliminarily mapped when present in 5-10% of metaphase cells examined; and as possibly mapped when present in 2-5% of metaphase cells examined, and wherein a positive signal is defined as a fluoroscence signals that are seen on both chromatids of a single chromosome at the same band position.

2. The method of claim 1, wherein said cDNA sequence is from about 1.2 Kb to about 3 Mb.

3. The method of claim 2, wherein said cDNA sequence has a length of < 2 Kb.

4. The use of a composition in the method according to any one of the preceding claims, wherein said composition comprises a plurality of reagents, wherein each reagent contains a nucleic acid sequence derived from the human genome, and wherein each individual reagent corresponds to a specific locus on a chromosomal band.

5. Use according to claim 4, wherein all reagents in the composition are derived from a single chromosome band.

6. Use according to claim 4, wherein all reagents in the composition are derived from a single chromosome.

7. Use according to claim 4, wherein said reagents comprise nucleic acid sequences derived from centromeres.

8. Use according to claim 4, wherein said reagents comprise nucleic acid sequences derived from telomeres.

9. Use according to claim 4, wherein all reagents in the composition comprise a cDNA contig.

10. Use according to claim 4, wherein all reagents in the composition are nonchimeric.

## Patentansprüche

1. Verfahren zur hochauflösenden Kartierung einer cDNA-Sequenz an einem Einzelbandenlocus auf einem menschlichen Chromosom, wobei das Verfahren Fluoreszenz-In-situ-Hybridisierung und Reverse-(R-)Banding umfasst und das Verfahren die Schritte umfasst:
(a) In-situ-Hybridisierung menschlicher Chromosomenausstriche, die vor der In-situ-Hybridisierung mit der cDNA-Sequenz mindestens zwei bis drei Wochen bei Raumtemperatur reifen gelassen werden, wobei die cDNA-Sequenz mit Biotin markiert wird,
(b) Kontaktieren hybridisierter Chromosomenausstriche mit mindestens einer Fluoreszenzmarkierung,
(c) Durchführen einer Gegenfärbung durch Post-in-situ-Hybridisierungsfärbung mit Chromomycin A3 und Distamycin A, und
(d) Nachweisen der Anwesenheit der cDNA-Sequenz und Bestimmen der Chromosomenbandenposition der cDNA-Sequenz, wobei die cDNA-Sequenz als kartiert angesehen wird, wenn in ≥10 % von 200 untersuchten Metaphasenzellen mindestens ein Chromosom an einem Einzelort signalpositiv ist; wobei die cDNA-Sequenz als vorläufig kartiert angesehen wird, wenn sie in 5 - 10 % der untersuchten Metaphasenzellen vorhanden ist, und als möglicherweise kartiert, wenn sie in 2 - 5 % der untersuchten Metaphasenzellen vorhanden ist; und wobei ein positives Signal als Fluoreszenzsignale definiert ist, die auf beiden Chromatiden eines Einzelchromosoms an derselben Bandenposition zu sehen sind.

2. Verfahren nach Anspruch 1, wobei die cDNA-Sequenz von ca. 1,2 Kb bis ca. 3 Mb reicht.

3. Verfahren nach Anspruch 2, wobei die cDNA-Sequenz eine Länge von <2 Kb aufweist.

4. Verwendung einer Zusammensetzung in dem Verfahren gemäß einem der vorhergehenden Ansprüche, wobei die Zusammensetzung eine Vielzahl von Reagenzien umfasst, wobei jedes Reagens eine vom menschlichen Genom hergeleitete Nucleinsäuresequenz enthält und jedes einzelne Reagens einem spezifischen Locus auf einer Chromosomenbande entspricht.

5. Verwendung gemäß Anspruch 4, wobei alle Reagenzien in der Zusammensetzung von einer einzelnen Chromosomenbande hergeleitet sind.

6. Verwendung gemäß Anspruch 4, wobei alle Reagenzien in der Zusammensetzung von einem Einzelchromosom hergeleitet sind.

7. Verwendung gemäß Anspruch 4, wobei die Reagenzien von Centromeren hergeleitete Nucleinsäuresequenzen umfassen.

8. Verwendung gemäß Anspruch 4, wobei die Reagenzien von Telomeren hergeleitete Nucleinsäuresequenzen umfassen.

9. Verwendung gemäß Anspruch 4, wobei alle Reagenzien in der Zusammensetzung ein cDNA-Contig umfassen.

10. Verwendung gemäß Anspruch 4, wobei alle Reagenzien in der Zusammensetzung nicht-chimär sind.

## Revendications

1. Procédé pour la cartographie à haute résolution d'une séquence d'ADNc à un locus à bande unique sur un chromosome humain, dans lequel ledit procédé comprend l'hybridation *in situ* avec fluorescence et le banding inverse (R), ledit procédé comprenant les étapes consistant à :
(a) hybrider *in situ* des longueurs de chromosome humain qui sont vieillies à température ambiante pendant au moins 2 à 3 semaines avant l'hybridation *in situ* avec ladite séquence d'ADNc où ladite séquence d'ADNc est biotinylée,
(b) mettre en contact les longueurs de chromosome avec au moins un marqueur fluorescent,
(c) effectuer une contre-coloration par coloration post-hybridation *in situ* avec la chromomycine A3 et la distamycine A, et
(d) détecter la présence de ladite séquence d'ADNc et déterminer la position de bande chromosomique de ladite séquence d'ADNc, où ladite séquence d'ADNc est considérée comme étant cartographiée lorsqu'au moins un chromosome est positif pour des signaux à un site unique supérieurs ou égaux à 10 % de 200 cellules en métaphase examinées ; où ladite séquence d'ADNc est considérée comme étant cartographiée de manière préliminaire lorsqu'elle est présente dans 5 à 10 % des cellules en métaphase examinées ; et comme étant potentiellement cartographiée lorsqu'elle est présente dans 2 à 5 % des cellules en métaphase examinées, et où un signal positif est défini en tant que signaux de fluorescence qui sont observés sur les deux chromatides d'un chromosome unique à la même position de bande.

2. Procédé selon la revendication 1, dans lequel ladite séquence d'ADNc est d'environ 1,2 Kb à environ 3 Mb.

3. Procédé selon la revendication 2, dans lequel ladite séquence d'ADNc a une longueur de < 2 Kb.

4. Utilisation d'une composition dans le procédé selon l'une quelconque des revendications précédentes, dans laquelle ladite composition comprend une pluralité de réactifs, où chaque réactif contient une séquence d'acide nucléique dérivée du génome humain, et dans laquelle chaque réactif individuel correspond à un locus spécifique sur une bande chromosomique.

5. Utilisation selon la revendication 4, dans laquelle tous les réactifs dans la composition sont dérivés d'une bande chromosomique unique.

6. Utilisation selon la revendication 4, dans laquelle tous les réactifs dans la composition sont dérivés d'un chromosome unique.

7. Utilisation selon la revendication 4, dans laquelle lesdits réactifs comprennent des séquences d'acide nucléique dérivées de centromères.

8. Utilisation selon la revendication 4, dans laquelle lesdits réactifs comprennent des séquences d'acide nucléique dérivées de télomères.

9. Utilisation selon la revendication 4, dans laquelle tous les réactifs dans la composition comprennent un contig d'ADNc.

10. Utilisation selon la revendication 4, dans laquelle tous les réactifs dans la composition sont non chimériques.
